(19) Europäisches Patentamt
European Patent Office
Office européen des brevets

(11) **EP 4 790 715 A1**

(12) **EUROPEAN PATENT APPLICATION**
published in accordance with Art. 153(4) EPC

(43) Date of publication:
**12.08.2026 Bulletin 2026/33**

(21) Application number: **24897516.1**

(22) Date of filing: **26.11.2024**

(51) International Patent Classification (IPC):
***G16C 60/00*** *(2019.01)* ***G06F 30/20*** *(2020.01)*
***G06F 113/26*** *(2020.01)*

(52) Cooperative Patent Classification (CPC):
**G06F 30/20; G16C 60/00;** G06F 2113/26

(86) International application number:
**PCT/JP2024/041723**

(87) International publication number:
**WO 2025/115831 (05.06.2025 Gazette 2025/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.11.2023 JP 2023200685**

(71) Applicant: **Sumitomo Riko Company Limited**
**Komaki-shi, Aichi 485-8550 (JP)**

(72) Inventors:
- **YAMAOKA, Natsuki**
  **Komaki-shi, Aichi 485-8550 (JP)**
- **WATANABE, Naoki**
  **Komaki-shi, Aichi 485-8550 (JP)**
- **HAYASHI, Takamitsu**
  **Komaki-shi, Aichi 485-8550 (JP)**
- **YAJIMA, Takashi**
  **Komaki-shi, Aichi 485-8550 (JP)**

(74) Representative: **Müller-Boré & Partner Patentanwälte PartG mbB**
**Friedenheimer Brücke 21**
**80639 München (DE)**

# (54) METHOD FOR CREATING SIMULATION MODEL OF COMPOSITE MATERIAL IN WHICH FILLER IS DISPERSED IN POLYMER, AND SIMULATION METHOD USING SAID SIMULATION MODEL

(57) A method for creating a simulation model of a composite material in which a filler is dispersed in a polymer, wherein a dispersion structure in a region adjacent to filler particle models is flexibly controllable. A method for creating, by a computer, a simulation model of the composite material includes: randomly placing filler particle models at coordinates that do not overlap each other in a model creation region; and newly placing a filler particle model in the model creation region in which filler particle models are already placed. The newly placing a filler particle model includes: setting coordinates of a filler particle model to be newly placed, based on coordinates of a filler particle model already placed and a preset distance between filler particle models; and newly placing a filler particle model at the set coordinates based on a preset upper particle coordination number.

FIG.2

S3
S31
SET COORDINATES OF FILLER PARTICLE MODEL TO BE NEWLY PLACED, BASED ON COORDINATES OF FILLER PARTICLE MODEL ALREADY PLACED
S32
IS PARTICLE COORDINATION NUMBER OF FILLER PARTICLE MODEL EQUAL TO OR LESS THAN UPPER PARTICLE COORDINATION NUMBER?
N
Y
S33
NEWLY PLACE FILLER PARTICLE MODEL AT COORDINATES SET IN S31
S4

EP 4 790 715 A1

## Description

### TECHNICAL FIELD

The present disclosure relates to a method for creating a simulation model of a composite material in which a filler is dispersed in a polymer, and a simulation method using the simulation model.

### BACKGROUND ART

The properties of a composite material containing a filler in a polymer vary depending on the dispersion form (morphology) of the filler. In the development of various composite materials, the effects of filler dispersion forms on various properties have been predicted by simulation. Since the accuracy of simulation depends largely on simulation models, several methods for creating models have been proposed.

For example, a method for creating a simulation model of a heterogeneous material has been proposed. The method includes a step of sequentially dispersing and generating particle models in a modeling region, wherein when the particle models are generated, the positions of the generated particle models are controlled such that the allowable value of the distance between centroids of a particle model already placed and a particle model to be newly generated decreases as the set number of particle models increases (JP-B-5854067).

### RELATED ART DOCUMENT

### PATENT DOCUMENT

JP-B-5854067

### SUMMARY

### PROBLEMS TO BE SOLVED BY THE DISCLOSURE

However, according to the study by the inventors of the present disclosure, the conventional method for creating a simulation model is insufficient for modeling a dispersion structure in a region adjacent to filler particles (primary particles), and it is difficult to flexibly control a short-distance network structure formed between filler particle models.

The present disclosure has been made in view of such circumstances and provides a novel method for creating a simulation model of a composite material in which a filler is dispersed in a polymer, wherein a dispersion structure in a region adjacent to filler particle models is flexibly controllable.

### MEANS FOR SOLVING THE PROBLEMS

The present disclosure has the following subject matters [1] to [9].

[1] A method for creating, by a computer, a simulation model of a composite material in which a filler is dispersed in a polymer, the method comprising:

randomly placing filler particle models at coordinates that do not overlap each other in a model creation region; and

newly placing a filler particle model in the model creation region in which filler particle models are already placed, wherein the newly placing a filler particle model comprises:

setting coordinates of a filler particle model to be newly placed, based on coordinates of a filler particle model already placed and a preset distance between filler particle models; and

newly placing a filler particle model at the set coordinates based on a preset upper particle coordination number.

[2] The method for creating a simulation model according to [1], wherein the newly placing a filler particle model is repeated until a preset filler filling rate is satisfied.

[3] The method for creating a simulation model according to [1] or [2], wherein the setting coordinates of a filler particle model to be newly placed comprises:

selecting an arbitrary filler particle model from the filler particle models already placed, and
setting, as the coordinates of a filler particle model to be newly placed, coordinates at which a distance between the coordinates of the selected arbitrary filler particle model and the coordinates of the filler particle model to be newly placed is the preset distance between filler particle models.

[4] The method for creating a simulation model according to any one of [1] to [3], wherein the newly placing a filler particle model at the set coordinates based on a preset upper particle coordination number comprises a newly placing a filler particle model at the set coordinates when a particle coordination number of a filler particle model in the model creation region is equal to or less than the preset upper particle coordination number.
[5] The method for creating a simulation model according to any one of [1] to [4], wherein the preset upper particle coordination number is three or four.
[6] The method for creating a simulation model according to any one of [1] to [5], wherein the preset upper particle coordination number is set by a formula:
upper particle coordination number = circumference ratio $\pi$ of filler particle model/(1 - [filler filling rate [volume %] $\times$ 0.01).
[7] The method for creating a simulation model according to any one of [1] to [6], wherein the preset distance between filler particle models is a preset distance between centroid coordinates of filler particle models, and the preset distance is 0.9 times or more and 1.0 times or less a diameter of the filler particle model.
[8] The method for creating a simulation model according to any one of [1] to [7], wherein the composite material is a composite material in which a conductive filler is dispersed in a polymer.
[9] A simulation method comprising an analyzing properties of a simulation model created by the method for creating a simulation model according to any one of [1] to [8].

EFFECTS OF THE DISCLOSURE

According to the present disclosure, the dispersion structure in a region adjacent to filler particle models is flexibly controllable.
The present disclosure enables flexible control of a short-distance network structure formed between filler particle models and therefore may contribute to improving the accuracy of simulation.
For example, according to an embodiment of the present disclosure, in a conductive property simulation for a composite material, the deviation between the tendency of volume resistance obtained by simulation and the tendency of volume resistance obtained by measurement of actual composite materials may be suppressed. This may contribute to improving the accuracy of simulation.
In this respect, the conventional conductive property simulation for a composite material fails to flexibly control the dispersion structure in a region adjacent to filler particle models, and it is difficult to create a simulation model that reflects the dispersion structure in the actual composite material. Therefore, the volume resistance obtained by simulation tends to deviate from the volume resistance obtained by measurement of actual composite materials. Specifically, for example, in a simulation in a condition with a low filler filling rate, it is difficult to reproduce the short-distance network structure formed between filler particles, and as a result, the volume resistance obtained by simulation tends to be higher than the measured value of the actual composite material. Thus, there is a problem in the accuracy of simulation. According to an embodiment of the present disclosure, the tendency for high resistance may be suppressed even in a simulation in a condition with a low filler filling rate. The embodiment of the present disclosure is therefore superior in that it may increase the accuracy of simulation.
In addition, in the conventional simulation method, even if an ideal filler dispersion structure is found, it is often difficult to reflect the ideal dispersion structure in the development of actual composite materials, because the simulation model includes composite elements. According to an embodiment of the present disclosure, it is possible to perform relatively simple modeling that takes into account the particleto-particle distance between filler particle models and the particle coordination number of filler particle models. Therefore, the embodiment of the present disclosure is also superior in that if an ideal filler dispersion structure is found from the simulation results, the ideal filler dispersion structure may be reflected in the development of actual composite materials relatively easily.

BRIEF DESCRIPTION OF DRAWINGS

FIG. 1 is a flowchart illustrating an example of a processing procedure of a simulation model creation method according to an embodiment of the present disclosure;
FIG. 2 is a flowchart illustrating an example of a processing procedure of the simulation model creation method

according to an embodiment of the present disclosure;

FIG. 3 is a flowchart illustrating an example of a processing procedure of the simulation model creation method according to an embodiment of the present disclosure;

FIG. 4 is a schematic illustration of the simulation model creation method according to an embodiment of the present disclosure;

FIG. 5 is a schematic illustration of the simulation model creation method according to an embodiment of the present disclosure;

FIG. 6 is a schematic illustration of the simulation model creation method according to an embodiment of the present disclosure;

FIG. 7 is a schematic illustration of the simulation model creation method according to an embodiment of the present disclosure;

FIG. 8 is a schematic illustration of the simulation model creation method according to an embodiment of the present disclosure;

FIG. 9 is a schematic illustration of the simulation model creation method according to an embodiment of the present disclosure;

FIG. 10 is a schematic illustration of the simulation model creation method according to an embodiment of the present disclosure;

FIG. 11 is a schematic illustration of the simulation model creation method according to an embodiment of the present disclosure;

FIG. 12 is a diagram illustrating an example of a simulation model obtained by the simulation model creation method according to an embodiment of the present disclosure; and

FIG. 13 is a diagram illustrating the results obtained by a simulation method according to an embodiment of the present disclosure.

EMBODIMENTS OF THE DISCLOSURE

A method for creating a simulation model according to an embodiment of the present disclosure (hereinafter also referred to as the present embodiment) is a method for creating, by a computer, a simulation model of a composite material in which a filler is dispersed in a polymer. The method is characterized in that the position of a filler particle model is controlled based on a preset distance between filler particle models, and that the particle coordination number of the filler particle model is controlled based on a preset upper particle coordination number. This enables flexible control of a dispersion structure in a region adjacent to filler particle models.

Embodiments of the present disclosure will be described in detail below.

In the present embodiment, the method for creating a simulation model and the simulation method are executed by a computer. The computer includes an information processing device such as a personal computer including a central processing unit (CPU), a ROM, a working memory, a storage device such as a magnetic disk, an input device such as a keyboard and a mouse, and a display device such as a display, wherein process routines stored in advance are executed by software and hardware working together, in the same manner as in conventional methods for creating simulation models.

In the present embodiment, the composite material to be modeled and analyzed is a composite material in which a filler is dispersed in a polymer.

Examples of the polymer include rubbers, resins, and elastomers, specifically, but not limited to, ethylene-propylene-diene monomer ternary copolymer (EPDM), acrylic rubber, urethane rubber, styrene-butadiene-styrene block polymer (SBS), styrene-isobutylene-styrene block polymer (SIBS), styrene-butadiene (SB) copolymer, styrene-isoprene (SI) copolymer, styrene-isoprene-styrene (SIS) copolymer, styrene-ethylene-butylene (SEB) copolymer, styrene-ethylene-butylene-styrene (SEBS) copolymer, styrene-ethylene-propylene (SEP) copolymer, styrene-ethylene-propylene-styrene (SEPS) copolymer, hydrogenated copolymers of the above copolymers, ethylene-propylene copolymer (EPR), butadiene rubber (BR), isoprene rubber (IR), styrene-butadiene rubber (SBR), liquid isoprene rubber (liquid IR), liquid butadiene rubber (liquid BR), liquid styrene-butadiene rubber (liquid SBR), liquid styrene-isoprene rubber (liquid SI), liquid styrene-ethylene-propylene rubber (liquid SEP), and liquid isoprenebutadiene rubber (liquid IR-BR).

Examples of the filler include carbon black, silica, talc, calcium carbonate, carbon fiber, and carbon nanotubes. Conductive fillers include conductive carbon fillers such as conductive carbon black, carbon nanotubes, and graphite.

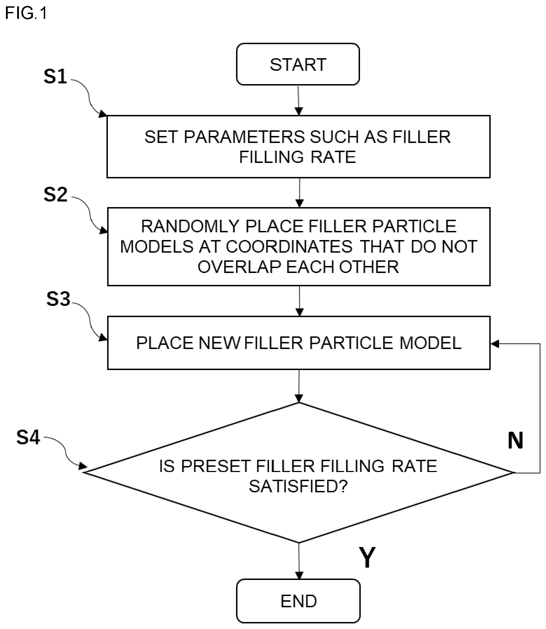

FIG. 1 to FIG. 3 are flowcharts illustrating an example of the main processing procedure of the method for creating a simulation model according to the present embodiment. The flowchart illustrates an exemplary embodiment. The method for creating a simulation model of the present disclosure is not limited in the order in the flowchart and the like.

<Step S1>

The method for creating a simulation model according to the present embodiment includes step S1 of setting a parameter such as a filler filling rate. In step S1, in addition to the filler filling rate, for example, the size of a filler particle model (radius, diameter, volume, etc.), the shape of the filler particle model (sphere, oblate sphere (long ellipsoid, oblate ellipsoid), plate-like shape, cylindrical shape, etc.), the range of a model creation region, the distance between filler particle models, a reference for calculating the particle coordination number of the filler particle model, an upper particle coordination number, and the like are preset.

<Step S2>

The method for creating a simulation model according to the present embodiment includes step S2 of randomly placing filler particle models at coordinates that do not overlap each other in the model creation region. In step S2, for example, a plurality of filler particle models corresponding to a subset of the total number of filler particle models corresponding to the filler filling rate set in step S1 above are randomly placed at coordinates at which the filler particle models do not overlap each other.

Specifically, in step S2, arbitrary coordinates in the model creation region are selected randomly using, for example, uniform random numbers, and filler particle models are sequentially placed at the selected coordinates. In the course of this processing, if the selected coordinates overlap with other filler particle models already placed, the selection of coordinates is performed again, and new coordinates are randomly selected. The processing in step S2 is terminated, for example, when a percentage or a number of filler particle models, out of the total number of filler particle models, is reached.

The number or the percentage of filler particle models to be placed in step S2 is preset according to the purpose of the simulation and the properties of the filler. For example, filler particle models as many as the number corresponding to about 0.3 to 20%, 1 to 10%, 1 to 5% of the total number corresponding to the filler filling rate are placed.

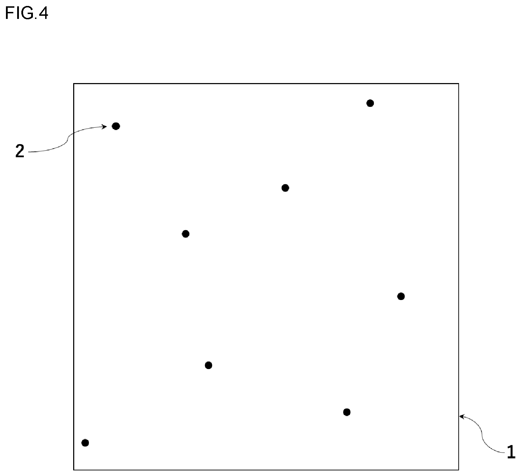

FIG. 4 is a diagram illustrating an example of the processing in step S2. For convenience, FIG. 4 shows a two-dimensional representation of a three-dimensional model creation region 1 and filler particle models 2, which are three-dimensional spheres (the same in the drawings subsequent to FIG. 4). Step S2 is, for example, a step of randomly placing a plurality of filler particle models 2 at coordinates that do not overlap each other in an initial state of the model creation region 1 (a state in which no filler particle models are present). As illustrated in FIG. 4, after execution of step S2, a plurality of filler particle models 2 are placed. The coordinates of the filler particle models 2 do not overlap each other, and the filler particle models 2 are placed at a distance from each other in a predetermined dispersion state.

The model creation region 1 is the entire region where a periodic boundary condition is defined, or a partial region having an arbitrary shape extracted from the region where the periodic boundary condition is defined, or a partial region having an arbitrary shape extracted from a space where the periodic boundary condition is not defined, in the same way as in a conventional simulation model. The range of the model creation region 1 is preset.

The filler particle model placed in the model creation region 1 is a model of primary particles of the filler. The shape of the filler particle model is generated by approximately modeling a shape such as sphere, oblate sphere (long ellipsoid, oblate ellipsoid), plate-like shape, or cylindrical shape. Parameters are predefined to specify the size (size of the region occupied in the model creation region) such as radius, diameter, and volume. In addition, in execution of various property simulations described below, necessary property parameters (e.g., conductivity) are set separately. In the present embodiment, a spherical filler particle model is set. However, filler particle models of other shapes may be used, and two or more shapes may be used in combination. In the present embodiment, the other region excluding the regions where the filler particle models are created is defined as a polymer model.

<Step S3>

The method for creating a simulation model according to the present embodiment includes step S3 of newly placing an additional filler particle model 3 in the model creation region 1 including a plurality of filler particle models 2 placed in step S2.

In step S3, the coordinates of the filler particle model 3 to be newly placed are controlled based on the filler particle models 2 already placed. For example, step S3 includes step S31 of setting the coordinates of the filler particle model 3 to be newly placed, based on the coordinates of the filler particle models 2 placed in step S2. In step S31, for example, an arbitrary filler particle model 2 is randomly selected from a plurality of filler particle models 2 already placed, based on a uniform function or the like (step S311). The selected filler particle model 2 serves as a reference or a starting point for generating the filler particle model 3 to be newly placed.

Next, based on the coordinates of the selected filler particle model 2 and a preset distance d between filler particle models, the coordinates of the filler particle model 3 to be newly placed are selected (step S312). Step S312 is a step of

controlling the relative coordinates between the coordinates of the filler particle model 3 to be newly placed and the coordinates of the selected filler particle model 2 serving as the starting point of the filler particle model 3 to be newly placed, among the filler particle models 2 already placed. Specifically, for example, the coordinates of the filler particle model 3 to be newly placed are randomly selected from a set of coordinates whose distance from the coordinates of the selected filler particle model 2 is the preset distance d between filler particle models, for example, using uniform random numbers. Such processing enables modeling that takes into account the filler dispersion structure in actual composite materials, for example, by minimizing the probability of isolation of the filler particle model.

The preset distance d between filler particle models may be set as appropriate according to the purpose of the simulation and the properties of the filler. For example, from the viewpoint of flexible control of the short-distance network, the preset distance d may be set based on an appropriate criterion, such as the distance at which the surface of the filler particle model 2 already placed and the surface of the filler particle model 3 to be newly placed come into contact with each other, the distance at which the surfaces of the filler particle models form a predetermined gap, or the distance at which the filler particle models share a predetermined overlapping region.

In an embodiment of the present disclosure, the preset distance d between filler particle models may be set as the distance between centroids of the filler particle models. The preset distance between filler particle models is, for example, 0.9 times or more and 1.5 times or less, 0.9 times or more and 1.0 times or less, the diameter of the filler particle model. Specifically, for example, the distance set by the following formula may be used.

(Formula) distance d between filler particle models = diameter of filler particle model $\times$ $\alpha$ (where $\alpha$ is 0.9 or more and 1.5 or less)

In an embodiment in which a conductive property simulation model is created, $\alpha$ in the formula is preferably less than 1.0, and preferably 0.9 or more and 0.95 or less.

Next, the step of selecting the coordinates of the filler particle model 3 to be newly placed based on the coordinates of the selected filler particle model 2 and the preset distance d between filler particle models will be described (steps S311 and S312).

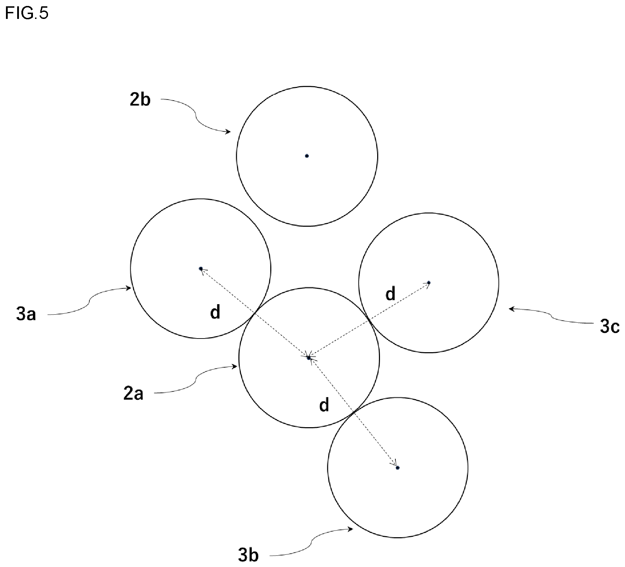

In FIG. 5 schematically illustrating filler particle models in a partial region of the model creation region 1, there are a plurality of filler particle models 2a, 2b... already placed. If a filler particle model 2a is randomly selected from a plurality of filler particle models 2a, 2b... already placed, arbitrary coordinates randomly selected from a set of coordinates at the preset distance d between filler particle models (in FIG. 5, the diameter of the filler particle models) from the centroid coordinates of the filler particle model 2a are set as the coordinates of the filler particle model 3 to be newly placed. Specifically, in FIG. 5, arbitrary coordinates randomly selected from the centroid coordinates of filler particle models 3a, 3b, 3c... are set as the coordinates of the filler particle model 3 to be newly placed. In other words, in FIG. 5, the filler particle models 3a, 3b, 3c... are the candidates for the filler particle model to be newly placed. Next, if each of the conditions described below is satisfied, the filler particle model 3 is newly placed at the set coordinates.

The same processing is then repeated in the present embodiment. In other words, filler particle models 3 are newly placed sequentially until the preset filler filling rate is satisfied, by repeating a series of processing of randomly selecting an arbitrary filler particle model 2 from a plurality of filler particle models 2... already placed, randomly setting arbitrary coordinates from a set of coordinates at a preset distance d between filler particle models from the centroid coordinates of the selected filler particle model 2, and if each of the conditions described below is satisfied, newly placing a filler particle model 3 at the set coordinates.

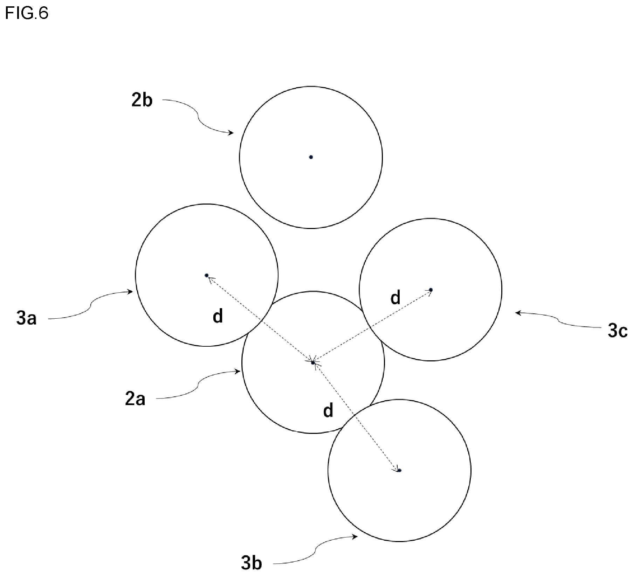

In FIG. 5 described above, the diameter of the filler particle model is set as the preset distance d between filler particle models, by way of example. However, as illustrated in FIG. 6, for example, when a conductive property simulation model is created, it is preferable that the distance d between filler particle models is set to a predetermined distance shorter than the diameter of the filler particle model (the distance at which the filler particle models slightly overlap).

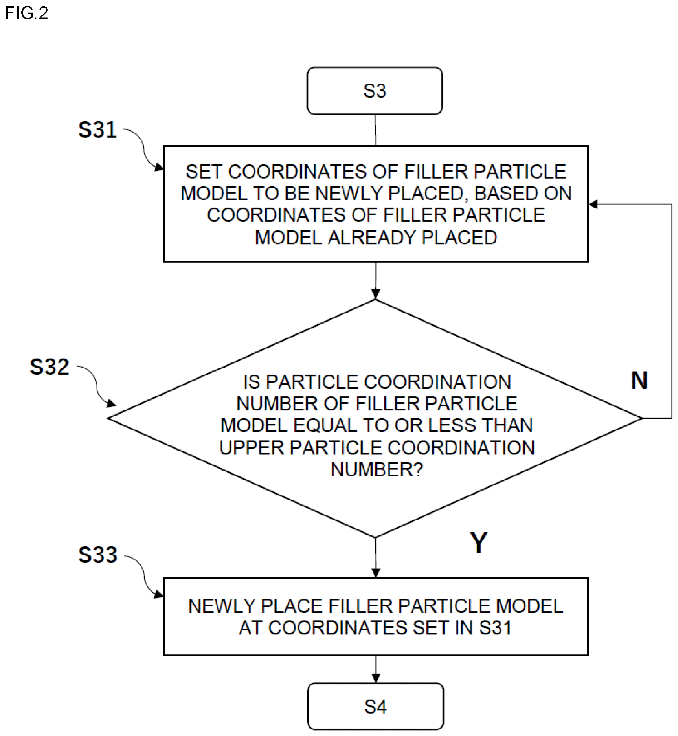

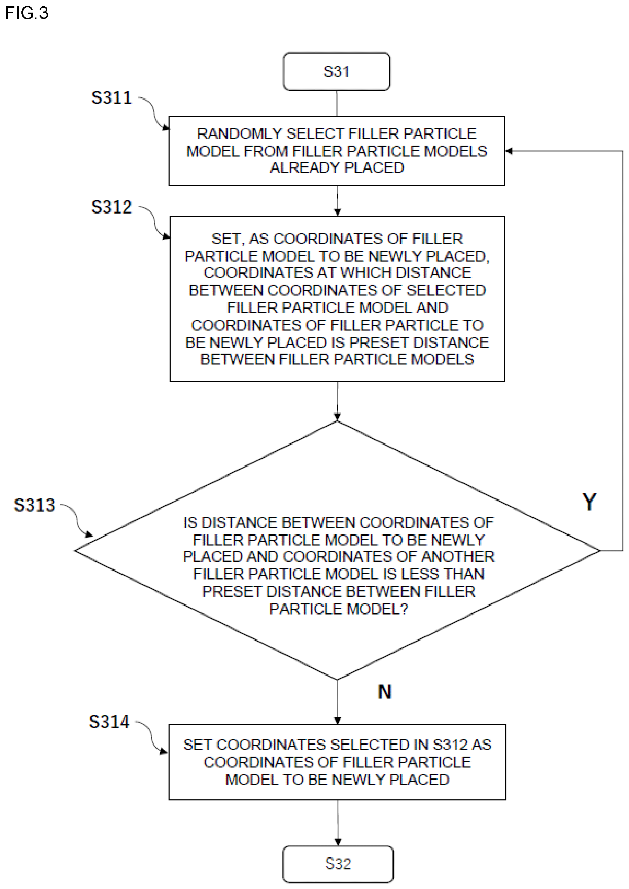

As described above, the coordinates of the filler particle model 3 to be newly placed are set based on the coordinates of the selected filler particle model 2 and the preset distance d between filler particle models, and if each of the following conditions is satisfied, the filler particle model 3 is newly placed at the set coordinates. In other words, in the method for creating a simulation model according to the present embodiment, as illustrated in step S313 of FIG. 3 and step S32 of FIG. 2, the conditions include: (1) the distance between the coordinates of the filler particle model 3 to be newly placed and the coordinates of another filler particle model is not less than the preset distance between filler particle models (the filler particle models do not excessively overlap each other); and (2) the particle coordination number of the filler particle model is equal to or less than a preset upper particle coordination number.

<Step S313>

First, step S313 is a step of controlling the relative coordinates between the coordinates of the filler particle model

to be newly placed and the coordinates of another filler particle model, excluding the filler particle model serving as the starting point of the filler particle model to be newly placed, among the filler particle models 2 already placed.

In other words, step S313 is a step for suppressing excessive overlap of the coordinates of the newly placed filler particle model with the coordinates of another filler particle model placed in the model creation region. Specifically, the relative coordinates to the filler particle model serving as the starting point of the filler particle model to be newly placed (selected filler particle model) among the filler particle models already placed are controlled by the preset distance d between filler particle models. Meanwhile, if the relative coordinates between the coordinates of the filler particle model to be newly placed and the coordinates of another filler particle model (unselected filler particle model), excluding the filler particle model serving as the starting point of the filler particle model to be newly placed (selected filler particle model), among the filler particle models 2 already placed, are not controlled, there is a possibility that the coordinates of the filler particle model to be newly placed and the coordinates of the other filler particle model (unselected filler particle models) excessively overlap. Such overlap is controlled in step S313.

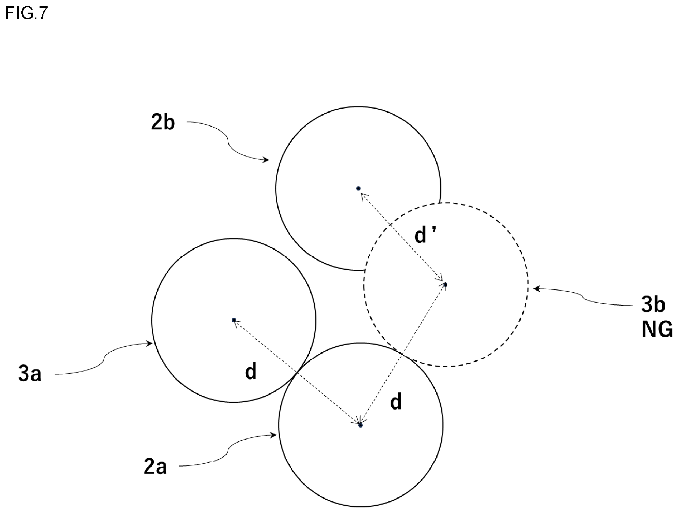

FIG. 7 is shown as an example. As illustrated in FIG. 7 schematically illustrating filler particle models in a partial region of the model creation region 1, there are a plurality of filler particle models 2a, 2b... already placed. If a filler particle model 2a is randomly selected from a plurality of filler particle models 2a, 2b... already placed, it is possible that arbitrary coordinates randomly selected from a set of coordinates at the preset distance d between filler particle models (in this embodiment, the diameter of the filler particle model) from the centroid coordinates of the filler particle model 2a are used as a candidate for coordinates of the filler particle model 3 to be newly placed. However, since the distance between the centroid coordinates of the filler particle model 3b and the centroid coordinates of the other filler particle model 2b is a distance d' which is less than the preset distance d between filler particle models (in the present embodiment, the diameter of the filler particle model), the centroid coordinates of the filler particle model 3b are not set as the coordinates of the filler particle model 3 to be newly placed.

As an example of the processing when the above condition is not satisfied, for example, an arbitrary filler particle model 2 is randomly selected again from a plurality of filler particle models 2 already placed, based on a uniform function or the like (step S311), and arbitrary coordinates randomly selected from a set of coordinates at the preset distance d between filler particle models (in the present embodiment, the diameter of the filler particle model) from the centroid coordinates of the selected filler particle model 2 are selected as a candidate for coordinates of the filler particle model 3 to be newly placed (step S312). Then, it is determined whether the above condition is satisfied (see FIG. 3). If the above condition is satisfied, the selected coordinates are newly set as the coordinates of the filler particle model.

In FIG. 7 described above, steps S312 and S313 are performed with reference to the preset distance d between filler particle models. However, the distance between filler particle models may be set differently depending on the purpose of the simulation or the like. For example, the distance between filler particle models in step S312, that is, the distance between the coordinates of the filler particle model 3 to be newly placed and the coordinates of the filler particle model 2 serving as the starting point, is set as a first distance d1 between filler particle models. On the other hand, the preset distance between filler particle models in step S313, that is, the distance between the coordinates of another filler particle model 2, excluding the filler particle model 2 serving as the starting point, and the coordinates of the filler particle model 3 to be newly placed, may be set individually as a second distance d2 between filler particle models.

<Step S32>

Next, <u>a step involving a condition</u> {a step of determining whether a condition is met} that the particle coordination number of the filler particle model is equal to or less than the upper particle coordination number (step S32) will be described. As described above, the coordinates of the filler particle model 3 to be newly placed are set based on the coordinates of the selected filler particle model 2 and the preset distance d between filler particle models. In the method for creating a simulation model according to the present embodiment, the step (step S32) <u>involving the condition</u> {of determining whether the condition is met} that the particle coordination number of the filler particle model is equal to or less than the preset upper particle coordination number is important. Specifically, this step <u>involves the condition</u> {determines whether the condition is met} that the particle coordination number of each of the selected filler particle model 2, the filler particle model 3 to be newly placed with the selected filler particle model 2 as a starting point, and the filler particle model 2 not selected is equal to or less than the preset upper particle coordination number.

The method for creating a simulation model according to the present embodiment includes a step of presetting the upper particle coordination number and a step of presetting a criterion for calculating the particle coordination number of the filler particle model.

The step of presetting the upper particle coordination number is a step of presetting the upper particle coordination number in consideration of the purpose of the simulation, filler properties, filler filling rate, and the like. The upper particle coordination number may be set, for example, based on empirical formulae in powder engineering. Specifically, for example, the upper particle coordination number may be preset according to the following formula.

(Formula) upper particle coordination number = circumference ratio $\pi$of filler particle model/(1 - [filler filling rate [volume %] $\times$ 0.01)

The preset upper particle coordination number is set as appropriate according to the purpose of the simulation and the filler properties. Specific examples of the preset upper particle coordination number are about 3 to 5 or 3 to 4 for a conductive property simulation model.

The step of presetting a criterion for determining the particle coordination number of the filler particle models is a step of presetting a criterion for determining how many filler particle models any given filler particle model is coordinated with in the model creation region.

As used herein, the particle coordination number is a concept in powder engineering that refers to the number of contact points on the surface of one particle with other particles. In the present disclosure, the criterion may be set as appropriate in consideration of the purpose of the simulation and the properties of the target filler. For example, in addition to setting the number of contact points on the surface of one filler particle model with other filler particle models as a criterion, for example, the number of other filler particle models in contact with one filler particle model may be set as a criterion.

Furthermore, even when one filler particle model is not geometrically in contact with another filler particle model, it may be appropriate to consider these filler particle models as being in contact if they are in close proximity, in consideration of the purpose of the simulation and the properties of the target filler. Therefore, the number of filler particle models having coordinates in a region surrounding one filler particle model may be set as a criterion. Specifically, for example, assuming a surrounding region formed when the volume of one filler particle model is expanded in a similar shape at an arbitrary magnification ratio around the centroid coordinates of the one filler particle model, the number of other filler particle models that have centroid coordinates in the surrounding region may be set as a criterion. Further, for example, assuming a surrounding region whose center is at the centroid coordinates of one filler particle model and whose radius is 1.0 to 1.5 times the diameter of the filler particle model, the number of other filler particle models having the centroid coordinates in the surrounding region may be set as a criterion.

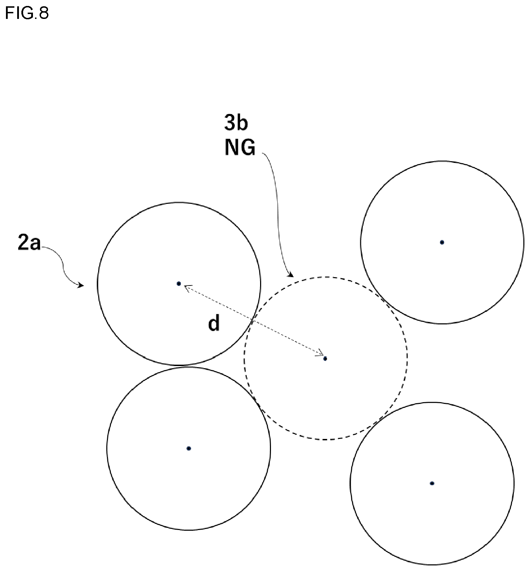

FIG. 8 is shown as an example. When the preset upper particle coordination number is three, and the criterion for determining the preset particle coordination number of the filler particle model is based on the number of other filler particle models in contact with one filler particle model, the particle coordination number of the filler particle model 3b is four in the arrangement in FIG. 8 schematically illustrating filler particle models in a partial region of the model creation region 1. Therefore, the centroid coordinates of the filler particle model 3b are not set as the coordinates of the filler particle model 3 to be newly placed. Specifically, in FIG. 8, with four filler particle models already existing, if the filler particle model 2a is randomly selected (step S311), and the centroid coordinates of the filler particle model 3b are set as the coordinates at the preset distance d between filler particle models (in the present embodiment, the diameter of the filler particle model) from the centroid coordinates of the filler particle model 2a, the particle coordination number of the filler particle model 3b will be four. Therefore, the centroid coordinates of the filler particle model 3b are not set as the coordinates of the filler particle model 3 to be newly placed.

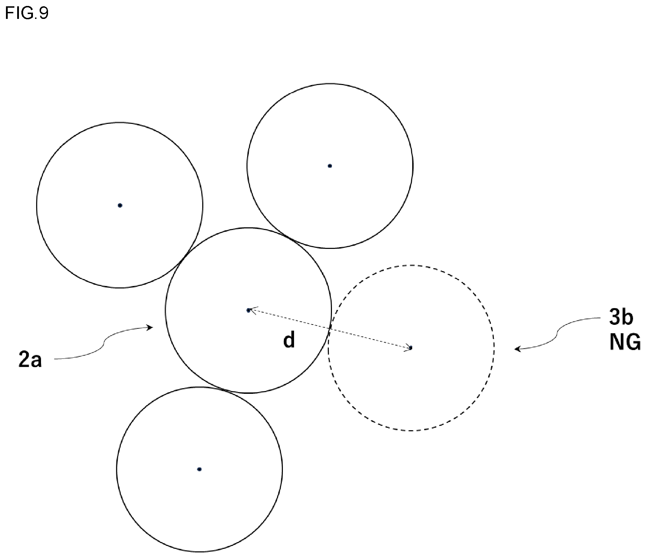

FIG. 9 is also shown as an example. When the preset upper particle coordination number is three, and the criterion for determining the preset particle coordination number of the filler particle model is based on the number of other filler particle models in contact with one filler particle model, the particle coordination number of the filler particle model 2a is four in the arrangement in FIG. 9 schematically illustrating filler particle models in a partial region of the model creation region 1. Therefore, the centroid coordinates of the filler particle model 3b are not set as the coordinates of the filler particle model 3 to be newly placed. Specifically, in FIG. 9, with four filler particle models already existing, if the filler particle model 2a is randomly selected (step S311), and the centroid coordinates of the filler particle model 3b are set as the coordinates at the preset distance d between filler particle models (in the present embodiment, the diameter of the filler particle model) from the centroid coordinates of the filler particle model 2a, the particle coordination number of the filler particle model 2a will be four. Therefore, the centroid coordinates of the filler particle model 3b are not set as the coordinates of the filler particle model 3 to be newly placed.

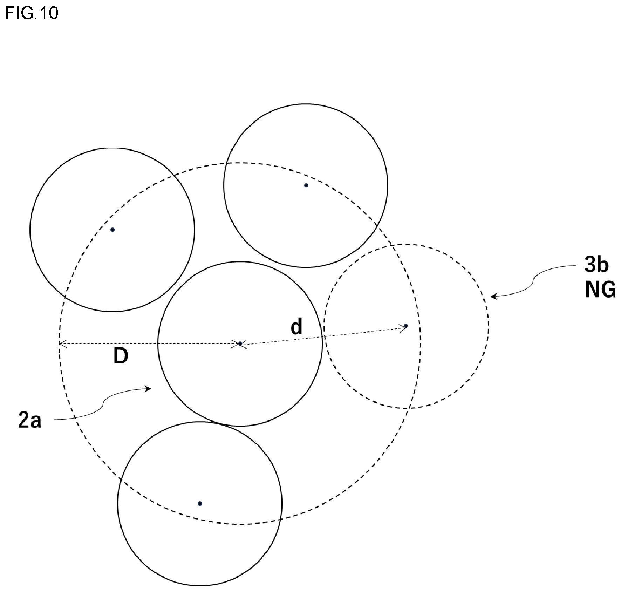

FIG. 10 is also shown as an example. When the preset upper particle coordination number is three, and the criterion for determining the preset particle coordination number of the filler particle model is based on the number of other filler particle models having the centroid coordinates in a surrounding region whose center is at the centroid coordinates of one filler particle model and whose radius is a distance (D) that is 1.1 times the diameter of the filler particle model, the particle coordination number of the filler particle model 2a is four in the arrangement in FIG. 10 schematically illustrating filler particle models in a partial region of the model creation region 1. Therefore, the centroid coordinates of the filler particle model 3b are not set as the coordinates of the filler particle model 3 to be newly placed. Specifically, in FIG. 10, with four filler particle models already existing, if the filler particle model 2a is randomly selected (step S311), and the centroid coordinates of the filler particle model 3b are set as the coordinates at the preset distance d between filler particle models (in the present embodiment, the diameter of the filler particle model) from the centroid coordinates of the filler particle

model 2a, the particle coordination number of the filler particle model 2a will be four. Therefore, the centroid coordinates of the filler particle model 3b are not set as the coordinates of the filler particle model 3 to be newly placed.

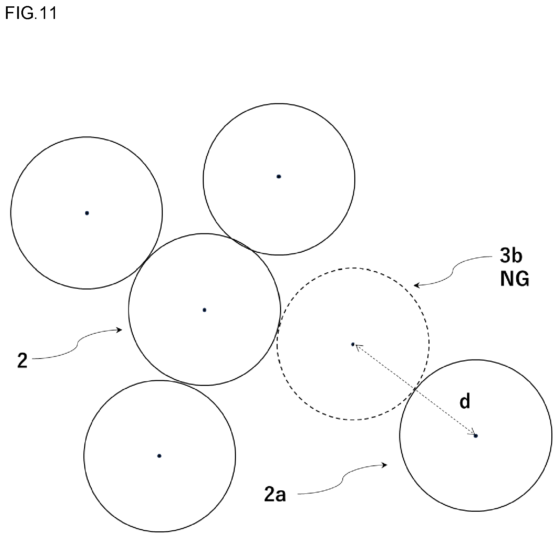

FIG. 11 is also shown as an example. When the preset upper particle coordination number is three, and the criterion for determining the preset particle coordination number of the filler particle model is based on the number of other filler particle models in contact with one filler particle model, the particle coordination number of the filler particle model 2 is four in the arrangement in FIG. 11 schematically illustrating filler particle models in a partial region of the model creation region 1. Therefore, the centroid coordinates of the filler particle model 3b are not set as the coordinates of the filler particle model 3 to be newly placed. Specifically, in FIG. 11, with five filler particle models already existing, if the filler particle model 2a is randomly selected (step S311), and the centroid coordinates of the filler particle model 3b are set as the coordinates at the preset distance d between filler particle models (in the present embodiment, the diameter of the filler particle model) from the centroid coordinates of the filler particle model 2a, the particle coordination number of the filler particle model 2 will be four. Therefore, the centroid coordinates of the filler particle model 3b are not set as the coordinates of the filler particle model 3 to be newly placed.

As an example of the processing when the condition on the upper particle coordination number is not satisfied, for example, an arbitrary filler particle model 2 is randomly selected again based on a uniform function or the like from a plurality of filler particle models 2 already placed (step S311), and the processing in steps S312 to S314 is performed. In addition, it is repeatedly determined whether the condition in step S32 is satisfied. If the condition in step S32 is satisfied, a filler particle model is newly placed at the coordinates selected in step S312.

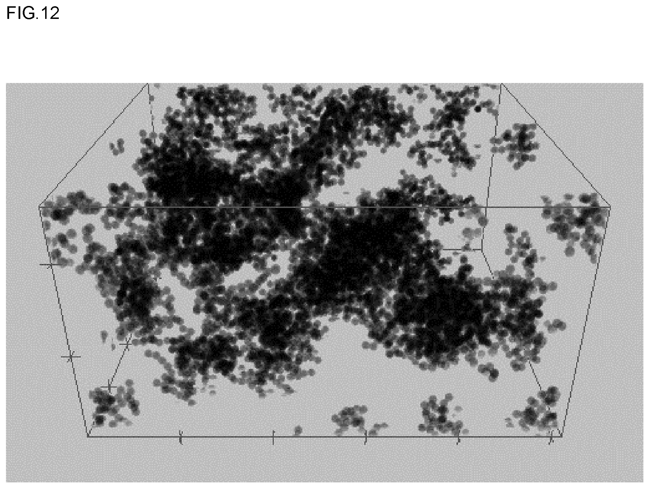

By repeating the above steps, filler particle models are newly placed sequentially, and if the preset filler filling rate is met, the simulation model creation is terminated (step S4: see FIG. 1). The simulation model obtained by the method for creating a simulation model according to the present embodiment is schematically illustrated in FIG. 12.

In the foregoing embodiment, for convenience of explanation, the condition in step S32 is determined after the condition in step S313 is determined. However, the order in which the conditions are determined is not limited, and the processing may be performed so that a filler particle model is newly placed when both conditions are met.

In an embodiment of the present disclosure, the simulation model may be used in various simulation methods. The simulation model may be used as a base model for simulation models used in various simulation methods. For example, the simulation model may be applied to create a simulation model to be used in a molecular dynamics simulation method.

In the embodiment of the present disclosure, the simulation model is a two-phase model of a filler particle model and a polymer model, but the simulation model is not limited to this and may include other model elements.

Furthermore, although the method for creating a simulation model according to the present embodiment has an advantage in that a simulation model may be flexibly created without using TEM images and the like obtained by observing actual composite materials, it is also possible to acquire images such as TEM images obtained by observing actual composite materials and to create a simulation model based on the acquired images.

<Simulation Method Using Simulation Model>

The simulation method including a step of analyzing the properties of the simulation model will be described using a conductive property simulation method as an example. However, the present disclosure is not limited to the conductive property simulation method, but may also be applied to, for example, a simulation method for mechanical properties such as durability.

The interface between the filler particle model and the polymer model created as described above is defined as an interfacial layer model. As an example of the interfacial layer model, for example, among the position elements of filler particle models, a position element with at least one adjacent element being a polymer model may be defined as an interfacial layer model.

Further, positive and negative electrode elements are added as electrode elements in the simulation model. The positive and negative electrode elements are models corresponding to electrodes with high conductivity. The positive electrode element is located at one end of the simulation model in the left-right direction, and the negative electrode element is located at the other end in the left-right direction. A predetermined positive electrode potential is set for the positive electrode element, and a predetermined negative electrode potential, for example, ground potential, is set for the negative electrode element.

The conductivity between adjacent position elements is then set based on a preset conductivity of the filler particle model, a preset conductivity of the polymer model, a preset conductivity of the interfacial layer model, a preset conductivity of the positive electrode element, and a preset conductivity of the negative electrode element. The conductivity between adjacent position elements is set to different conductivities depending on the type of adjacent position elements. In this way, a simulation model for conductive property analysis is created.

Analysis of conductive properties may be performed in accordance with conventionally known methods. For example, as input conditions, a predetermined positive electrode potential is applied to the positive electrode element and

a predetermined negative electrode potential is applied to the negative electrode element, and analysis of electrical resistance between the positive electrode element and the negative electrode element is performed. It is also possible to analyze a conductive path or the like based on a current value for each position element.

## EXAMPLES

A simulation model SM1 was created using the method for creating a simulation model according to the above embodiment, and conductive property simulations were performed. Various parameters are as follows.

In the simulation model SM1, the preset upper particle coordination number is a value set based on the formula (circumference ratio $\pi$of filler particle model / (1 - [filler filling rate [volume %] $\times$ 0.01). The criterion for determining the preset particle coordination number of the filler particle model is based on the number of other filler particle models having the centroid coordinates in a surrounding region whose center is at the centroid coordinates of one filler particle model and whose radius is 1.0 times the diameter of the filler particle model (see, for example, FIG. 10).

(Parameters)

Radius of filler particle model: 5 voxels
Model creation region: 512 $\times$ 512 $\times$ 256 $voxel^3$
Distance between filler particle models: diameter of filler particle model (10 voxels) $\times$ 0.93
Upper particle coordination number: 3
Filling rate of filler particle model: 5.0 vol%
Resistance of filler particle model: $10^{-2}$ $\Omega \cdot cm$
Resistance of polymer model: $10^{12}$ $\Omega \cdot cm$
Resistance of interfacial layer model: 10° $\Omega \cdot cm$
Initial condition: potential 0 for all voxels (electrodes are each 2 voxels thick)
Convergence condition: change in total potential sum < $10^{0}$ or 1 million loops

A simulation model SM2 was created in the same way as the simulation model SM1, except that the upper particle coordination number was not set (no limit in the upper particle coordination number), and conductive property simulations were performed.

<Evaluation of Particle Coordination Number>

Since the simulation model SM2 was created with no upper particle coordination number, the number of filler particle models with a particle coordination number of four or more (specifically, particle coordination number of four to nine) was approximately 1000 (out of a total number of approximately 6400 filler particle models), whereas the simulation model SM1 was created with a limited upper particle coordination number, all filler particle models had a particle coordination number of three or less, and there were virtually no filler particle models with a particle coordination number of zero.

<Evaluation of Conductive Property Simulation>

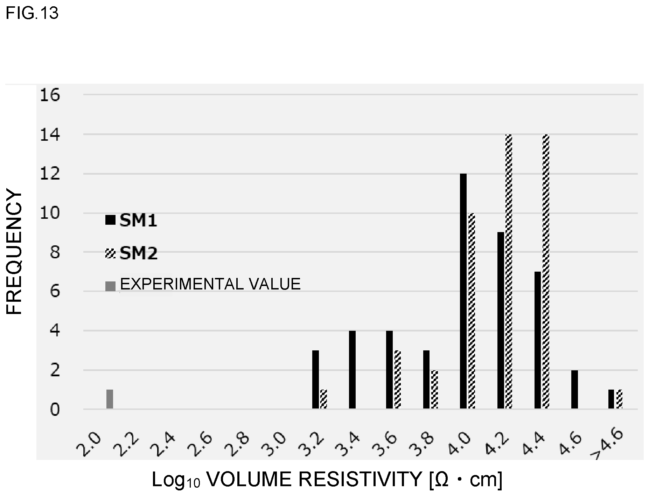

In conventional conductive property simulations, it is difficult to reproduce a short-distance network structure formed between filler particles. In particular, in a condition with a low filler filling rate (e.g., 5 to 8% or less), the volume resistance obtained from simulations tends to be higher than the measured value of actual composite materials. Thus, the conventional conductive property simulations have a problem in the accuracy of simulation. As a result of conductive property simulations using the simulation model SM1 (with a limited upper particle coordination number) or the simulation model SM2 (with no limit in the upper particle coordination number), it was confirmed that the volume resistance obtained using the simulation model SM1 was significantly lower than that of the simulation model SM2. Specifically, as illustrated in FIG. 13, the volume resistivity ($\Omega \cdot cm$) of the simulation model SM1 was confirmed to be significantly lower and closer to the measured value, compared with the volume resistivity ($\Omega \cdot cm$) of the simulation model SM2. The sample size N of each simulation model is 45.

In the above example, specific forms in the present disclosure are shown. The above example is merely illustrative and is not to be construed as limiting. Various modifications apparent to those skilled in the art are contemplated to be within the scope of the present disclosure.

INDUSTRIAL APPLICABILITY

The present disclosure provides a novel method for creating a simulation model of a composite material in which a filler is dispersed in a polymer, and is particularly useful, for example, as a method for creating a simulation model for use in conductivity simulation methods.

REFERENCE SIGNS LIST

1 model creation region
2 (2a...) filler particle model
3 (3a...) filler particle model (filler particle model to be newly placed)

## Claims

1. A method for creating, by a computer, a simulation model of a composite material in which a filler is dispersed in a polymer, the method comprising:

   randomly placing filler particle models at coordinates that do not overlap each other in a model creation region; and
   newly placing a filler particle model in the model creation region in which filler particle models are already placed, wherein the newly placing a filler particle model comprises:

      setting coordinates of a filler particle model to be newly placed, based on coordinates of a filler particle model already placed and a preset distance between filler particle models; and
      newly placing a filler particle model at the set coordinates based on a preset upper particle coordination number.

2. The method for creating a simulation model according to claim 1, wherein the newly placing a filler particle model is repeated until a preset filler filling rate is satisfied.

3. The method for creating a simulation model according to claim 1 or 2, wherein the setting coordinates of a filler particle model to be newly placed comprises:

   selecting an arbitrary filler particle model from the filler particle models already placed; and
   setting, as the coordinates of a filler particle model to be newly placed, coordinates at which a distance between the coordinates of the selected arbitrary filler particle model and the coordinates of the filler particle model to be newly placed is the preset distance between filler particle models.

4. The method for creating a simulation model according to any one of claims 1 to 3, wherein the newly placing a filler particle model at the set coordinates based on a preset upper particle coordination number comprises newly placing a filler particle model at the set coordinates when a particle coordination number of a filler particle model in the model creation region is equal to or less than the preset upper particle coordination number.

5. The method for creating a simulation model according to any one of claims 1 to 4, wherein the preset upper particle coordination number is three or four.

6. The method for creating a simulation model according to any one of claims 1 to 5, wherein the preset upper particle coordination number is set by a formula:

   upper particle coordination number = circumference ratio $\pi$of a filler particle model / (1 - [filler filling rate [volume %] $\times$ 0.01).

7. The method for creating a simulation model according to any one of claims 1 to 6, wherein the preset distance between

filler particle models is a preset distance between centroid coordinates of filler particle models, and the preset distance is 0.9 times or more and 1.0 times or less a diameter of the filler particle model.

8. The method for creating a simulation model according to any one of claims 1 to 7, wherein the composite material is a composite material in which a conductive filler is dispersed in a polymer.

9. A simulation method comprising analyzing properties of a simulation model created by the method for creating a simulation model according to any one of claims 1 to 8.

FIG.1

START
S1
SET PARAMETERS SUCH AS FILLER FILLING RATE
S2
RANDOMLY PLACE FILLER PARTICLE MODELS AT COORDINATES THAT DO NOT OVERLAP EACH OTHER
S3
PLACE NEW FILLER PARTICLE MODEL
S4
IS PRESET FILLER FILLING RATE SATISFIED?
N
Y
END

FIG.2

S3
S31
SET COORDINATES OF FILLER PARTICLE MODEL TO BE NEWLY PLACED, BASED ON COORDINATES OF FILLER PARTICLE MODEL ALREADY PLACED
S32
IS PARTICLE COORDINATION NUMBER OF FILLER PARTICLE MODEL EQUAL TO OR LESS THAN UPPER PARTICLE COORDINATION NUMBER?
N
Y
S33
NEWLY PLACE FILLER PARTICLE MODEL AT COORDINATES SET IN S31
S4

FIG.3

S31
S311
RANDOMLY SELECT FILLER PARTICLE MODEL FROM FILLER PARTICLE MODELS ALREADY PLACED
S312
SET, AS COORDINATES OF FILLER PARTICLE MODEL TO BE NEWLY PLACED, COORDINATES AT WHICH DISTANCE BETWEEN COORDINATES OF SELECTED FILLER PARTICLE MODEL AND COORDINATES OF FILLER PARTICLE TO BE NEWLY PLACED IS PRESET DISTANCE BETWEEN FILLER PARTICLE MODELS
S313
IS DISTANCE BETWEEN COORDINATES OF FILLER PARTICLE MODEL TO BE NEWLY PLACED AND COORDINATES OF ANOTHER FILLER PARTICLE MODEL IS LESS THAN PRESET DISTANCE BETWEEN FILLER PARTICLE MODEL?
Y
N
S314
SET COORDINATES SELECTED IN S312 AS COORDINATES OF FILLER PARTICLE MODEL TO BE NEWLY PLACED
S32

FIG.4

2
1

FIG.5

2b
3a
d
d
3c
2a
d
3b

FIG.6

2b
3a
d
d
3c
2a
d
3b

FIG.7

2b
d'
3b
NG
3a
d
d
2a

FIG.8

3b
NG
2a
d

FIG.9

2a
d
3b
NG

FIG.10

3b
NG
d
D
2a

FIG.11

3b
NG
2
d
2a

FIG.12

FIG.13

16
14
12
10
8
6
4
2
0
FREQUENCY
SM1
SM2
EXPERIMENTAL VALUE
2.0
2.2
2.4
2.6
2.8
3.0
3.2
3.4
3.6
3.8
4.0
4.2
4.4
4.6
>4.6
Log10 VOLUME RESISTIVITY [Ω・cm]

## INTERNATIONAL SEARCH REPORT

International application No.
**PCT/JP2024/041723**

**A. CLASSIFICATION OF SUBJECT MATTER**

***G16C 60/00***(2019.01)i; ***G06F 30/20***(2020.01)i; *G06F 113/26*(2020.01)n
FI: G16C60/00; G06F30/20; G06F113:26

According to International Patent Classification (IPC) or to both national classification and IPC

**B. FIELDS SEARCHED**

Minimum documentation searched (classification system followed by classification symbols)

G06Q10/00-99/00; G16C10/00-99/00; G16Z99/00; G06F30/00-30/398; G06F113/26

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2020-177375 A (THE YOKOHAMA RUBBER CO., LTD.) 29 October 2020 (2020-10-29) paragraphs [0025], [0029]-[0040] | 1-9 |
| A | JP 2015-79450 A (SUMITOMO RUBBER INDUSTRIES, LTD.) 23 April 2015 (2015-04-23) paragraphs [0039]-[0051] | 1-9 |
| A | JP 2016-9458 A (SUMITOMO RUBBER INDUSTRIES, LTD.) 18 January 2016 (2016-01-18) paragraphs [0023]-[0038], [0064]-[0069] | 1-9 |
| A | JP 2017-162331 A (THE YOKOHAMA RUBBER CO., LTD.) 14 September 2017 (2017-09-14) paragraphs [0023]-[0033], [0036]-[0039], [0045]-[0046] | 1-9 |
| A | JP 2016-71519 A (TOYO TIRE & RUBBER CO., LTD.) 09 May 2016 (2016-05-09) paragraphs [0015]-[0024], [0030]-[0033] | 1-9 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 February 2025** | **18 February 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No. PCT/JP2024/041723

C. DOCUMENTS CONSIDERED TO BE RELEVANT

| Category* | Citation of document, with indication, where appropriate, of the relevant passages | Relevant to claim No. |
|---|---|---|
| A | CN 115034061 A (BEIJING UNIVERSITY OF TECHNOLOGY) 09 September 2022 (2022-09-09) paragraphs [0001], [0039]-[0046] | 1-9 |

Form PCT/ISA/210 (second sheet) (July 2022)

**INTERNATIONAL SEARCH REPORT**
**Information on patent family members**

International application No.
**PCT/JP2024/041723**

| Patent document cited in search report | Publication date (day/month/year) | Patent family member(s) | Publication date (day/month/year) |
|---|---|---|---|
| JP 2020-177375 A | 29 October 2020 | (Family: none) | |
| JP 2015-79450 A | 23 April 2015 | (Family: none) | |
| JP 2016-9458 A | 18 January 2016 | (Family: none) | |
| JP 2017-162331 A | 14 September 2017 | (Family: none) | |
| JP 2016-71519 A | 09 May 2016 | (Family: none) | |
| CN 115034061 A | 09 September 2022 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5854067 B